# EUROPÄISCHE PATENTSCHRIFT

(11) **EP 3 018 599 B1**
(45) Veröffentlichungstag und Bekanntmachung des Hinweises auf die Patenterteilung: **15.04.2020**
(21) Anmeldenummer: 14191825.0
(22) Anmeldetag: 05.11.2014
(51) Int. Cl.: G16H 40/63

(54) **Übertragen von Darstellungszustandsinformationen sowie zugehörige Kommunikationseinrichtungen**
Transmission of presentation state and associated communication devices
Transfert d'informations concernant l'état de représentation d'une image et dispositifs de communications correspondants

(43) Veröffentlichungstag der Anmeldung: 11.05.2016
(73) Patentinhaber: Siemens Healthcare GmbH, 91052 Erlangen (DE)
(72) Erfinder: Eckl, Roland, 91301 Forchheim (DE); Spitzner, Christian, 91301 Forchheim (DE)

(56) Entgegenhaltungen:
- WO-A2-2008/084330
- US-A1- 2009 125 816
- YANAGIDA T ET AL: "Architecture for migratory adaptive user interfaces", COMPUTER AND INFORMATION TECHNOLOGY, 2008. CIT 2008. 8TH IEEE INTERNATIONAL CONFERENCE ON, IEEE, PISCATAWAY, NJ, USA, 8. Juli 2008 (2008-07-08), Seiten 450-455, XP031302664, ISBN: 978-1-4244-2357-6

## Beschreibung

### Hintergrund der Erfindung

Die Erfindung betrifft eine Kommunikationsanordnung nach dem Oberbegriff des Anspruchs 1 sowie ein Verfahren nach dem Oberbegriff des Anspruchs 8. Eine solche Kommunikationseinrichtung und ein solches Verfahren sind aus T. Yanagida, H. Nonaka, "Architecture for Migratory Adaptive User Interfaces", 8th Conference on Computer and Information Technology (CIT), 2008, Piscataway (IEEE), S. 450-455 bekannt.

Aus WO 2008/084330 A2 ist die Übertragung von Presentation States nach dem DICOM-Standard bekannt.

Ein Verfahren zur Übertragung von Bilddarstellungen zwischen Bedienschnittstellen ist schließlich aus US 2009/0125816 A1.

Die Erfindung liegt insbesondere auf den Gebieten der Medizintechnik und der medizinischen Informatik. Es sind auch andere Anwendungen wie z.B. in der industriellen Fertigung denkbar.

In medizinischen Geräten wie z.B. C-Bogen-Röntgengeräte, CT- bzw. MRT-Geräten kommen vermehrt grafische Bedienschnittstellen an Arbeitsplatzrechnern mit touchbasierter Eingabe zum Einsatz. Diese werden genutzt, um das Gerät zu bedienen und gewonnene Daten wie z.B. medizinische Bilddaten anzuzeigen. Zur Erhöhung des Bedienkomforts kann ein medizinisches Gerät mit mehreren, räumlich verteilten Arbeitsplatzrechner mit Bedienpanels bzw. Bildschirmen in Verbindung stehen. Dies ermöglicht den Nutzern, das Gerät von verschiedenen Positionen aus zu bedienen. Dabei erwartet der Nutzer, dass die Bedienpanels sich identisch verhalten und jeweils den aktuellen Systemzustand widerspiegeln.

Die Software eines Bedienpanels wird in eine Basissoftware, die Gerätetreiber und Betriebssystem beinhaltet, und eine Applikationssoftware unterteilt, die die für das Gerät spezifische Anwendung inklusive Bedienschnittstelle (GUI - Graphical User Interface) realisiert.

Die Bedienpanels benutzen die gleiche Applikationssoftware und die Bedienschnittstellen der verschiedenen Bedienpanels werden mittels entsprechender Nachrichten über ein gemeinsames Netzwerk synchronisiert.

In diesem Zusammenhang ist folgendes Szenario möglich: Ein Nutzer arbeitet an einem solchen Arbeitsplatzrechner. Dort werden während seiner Tätigkeiten von unterschiedlichen Modalitäten (z.B. Computertomographen (CT)-/Magnetresonanz (MR)-/Molekular-Imaging (MI)-Scanner etc.) erhaltene Ausgangsbilder zur Befundung auf verschiedene Visualisierungsarten (sog. Filtern) dargestellt. Dabei werden die Parameter für die verschiedenen Ansichten vom Nutzer angepasst (z.B. Fensterung auf 2D-Bildern), um bestimmte Bereiche herauszuarbeiten und Markierungen und/oder Messungen zu erzeugen. Diese können in abgeleiteten Bildern oder speziellen DICOM-Objekten, (z.B. Presentation States - Präsentationsstatus bzw. Darstellungszustand) gespeichert und für die Wiederherstellung der Ansicht verwendet werden.

Nun soll der Nutzer auf einem mobilen Gerät (oder einem anderen Arbeitsplatzrechner) weiterarbeiten. Der aktuelle Zustand der Darstellung soll dabei auf ein mobiles Gerät übertragen werden, um ggf. auf einem solchen mobilen Gerät z.B. ein Smartphone, Phablet bzw. Tablet weiterzuarbeiten. Auch ist es möglich, zur Befundungsdurchsprache mit dem Patienten bzw. zu Konsultationen mit Kollegen den aktuellen Zustand auf einem anderen mobilen Gerät (z.B. Smartphone) mitzunehmen.

Der Zustand bzw. Status kann nachgelagert auch auf ein weiteres mobiles Gerät oder auch einen stationären Arbeitsplatzrechner weitergereicht werden.

Eine korrekte, möglichst verzögerungsfreie und synchronisierte bzw. identische Anzeige an allen Bedienpanels ist bei medizinischen Geräten besonders wichtig, um Fehler bei der Befundung bzw. eine Fehlbedienung/-steuerung zu verhindern.

Als mobile Geräte bzw. Mobilgeräte sollen vorzugsweise marktübliche Standardgeräte, basierend auf den gängigen bzw. zukünftigen Betriebssystemen z.B. iOS-, Android-, Windows-, Fire-, Mozilla-Geräte, usw.) verwendet werden, die vorzugsweise keine Spezialhardware bzw. -software benötigen.

Dieses Vorgehen erfordert bei der Implementierung der Applikationssoftware einen relativ hohen Aufwand, da alle Bedieneingaben - z.B. das Betätigen eines Buttons - an einem Bedienpanel individuell bearbeitet und an alle anderen Bedienpanel über die genannten Nachrichten verteilt werden müssen. Außerdem muß für verschiedene Applikationssoftwarepakete diese Art der Mehrfach-Panel-Bedienung neu implementiert werden.

In der Nutzung von mehreren Bedienpanels liegt für den Nutzer oft der Nachteil, dass Daten über mehrere Eingabemasken bzw. Bedienschnittstellen manuell eingegeben werden müssen. Diese Daten müssen oft aus dem Gedächtnis des Nutzers abgerufen oder von Dokumenten abgelesen und wieder eingegeben werden.

Mobile Geräte können zwar den Eingabeprozess unterstützen, sind jedoch nicht nahtlos in den Arbeits- bzw. Handlungsablauf (Workflow) integriert. Es ist möglich, eine Synchronisation zwischen mehreren Bedienpanels mit Hilfe der Bedienpanel-seitigen bzw. Client-seitigen GUI-Software (GUI= graphical user interface) und der Server-seitigen GUI-Software - wie oben bereits beschrieben - sicherzustellen. Jedoch haben mobile Geräte wie Tablet-PCs oder Smartphones in der Regel keine so aufwendige GUI-Software bzw. Intelligenz wie Bedienpanels zum Abgleich von GUI-Daten.

Ein Format für die Ablage der Informationen, die notwendig sind, eine aktuelle Ansicht bzw. Darstellung auf Basis derselben Ausgangsdaten (z.B. medizinische Bilddaten wie DICOM Images usw.) auf einem Bildschirm wiederherzustellen, ist bereits durch sogenannte Presentation States (bzw. Extended Presentation States) des bekannten DICOM-Standards (DICOM = Digital Imaging and Communications in Medicine) abgedeckt.

Applikations- bzw. Hersteller-spezifische Informationen, die nicht von DICOM (Extendend) Presentation States abgedeckt werden, können dort weiterhin als proprietäre Attribute eingebracht werden. Dies erlaubt, auch Darstellungen wiederherzustellen, die auf Daten und Bearbeitungsschritten beruhen die nicht explizit vom Standard abgedeckt sind.

Das Abspeichern eines Zustands/Status wird in der Regel manuell auf einem Arbeitsplatzrechner angestoßen und auf einem anderen Gerät (z.B. mittels einer App) wiederum manuell geladen. Die hierzu mehreren manuellen Schritte und Nutzerinteraktionen (z.B. für die Suche des gewünschten Presentation States für einen bestimmten Patienten) sind umständlich, langwierig und wenig benutzerfreundlich.

Die Aufgabe der vorliegenden Erfindung besteht darin, das Zusammenwirken hinsichtlich einer synchronisierten Darstellung von Daten auf verschiedenen Geräten, z.B. Arbeitsplatzrechnern und mobilen Geräten, mit minimalem Aufwand zu verbessern.

### Darstellung der Erfindung

Die Aufgabe wird erfindungsgemäß gelöst durch eine Kommunikationsanordnung mit den Merkmalen des Anspruchs 1 sowie durch ein Verfahren mit den Merkmalen des Anspruchs 8. Vorteilhafte Ausgestaltungen der Erfindung sind Gegenstand der abhängigen Patentansprüche oder lassen sich aus der nachfolgenden Beschreibung sowie den Ausführungsbeispielen entnehmen.

Die Kommunikationseinrichtung kann hierbei als mobiles Gerät ausgebildet sein. Zur Initiierung der Erzeugung der einen Darstellungsinformation bzw. von Darstellungszustandsinformationen kann/können ein oder mehrere sogenannte Tags verwendet werden.

Die Kommunikationseinrichtung, z.B. ein mobiles Gerät, kann selbst das Erzeugen von Darstellungszustandsinformationen, z.B. in Form eines solchen oben genannten DICOM Presentation States (abgekürzt als PS bezeichnet), ggf. auf einer anderen Kommunikationseinrichtung, z.B. einem Arbeitsplatzrechner, initiieren, um den Zustand der Darstellung von Daten wiederherzustellen.

Um die Erzeugung eines DICOM Presentation States zu initiieren, kommen sowohl passive sogenannte Tags wie QR-Codes und diverse graphische 2D-/3D-Codes, aber auch passive RFID- oder NFC-Tags und vergleichbares in Frage. Auch sind aktive Tags (aktive RFID- oder NFC-Tags und vergleichbares) einsetzbar. Als Tags werden auch Meta- und Zusatzinformationen bezeichnet, die einer Datei angefügt werden.

Das Auslesen solcher Tags wird i.d.R. von den meisten aktuellen mobilen Geräten ohne Zusatzhardware unterstützt. Insbesondere QR-Codes (QR = Quick Response), die mittels einer Erfassungseinheit z.B. einer Kamera erfasst werden, bzw. NFC-Tags, die mittels integrierten Lesegeräten erfasst werden, können hier zum Einsatz kommen.

Aktive Tags bei RFID (Radio Frequency IDentification) oder NFC (Near Field Communication) besitzen im Gegenteil zu passiven Tags den Unterschied, dass sie die Daten in vordefinierten Zeitintervallen senden können. Es werden kleine Transponder verwendet, die die Informationen vom RFID- bzw. NFC-Tag zum RFID- bzw. NFC-Lesegerät bzw. -Empfänger senden.

Aufgrund ihrer eigenen Stromversorgung sind sie nicht auf die übertragene Energie des Lesegerätes angewiesen. Passive Tags sind daher i.d.R. nicht mit einem Gerät/System verbunden und halten somit statische Daten vor.

Die Darstellungszustandsinformationen bzgl. der Darstellung der Daten kann in einer Speicherstelle bzw. in einem Speicher festgeschrieben werden können.

In einer Weiterbildung der Erfindung ist an der zweiten Kommunikationseinrichtung eine die Speicherstelle adressierbare Darstellungsidentifikationsinformation erhältlich.

In einer Weiterbildung der Erfindung können die Darstellungszustandsinformationen ggf. samt Daten mittels einer die Darstellungsidentifikationsinformation (ID) umfassenden Zugriffsinformation abrufbar sein. Um dies zu bewerkstelligen, kann ein Dienst bzw. Service verwendet werden, um die Zugriffsinformation ggf. einer anderen Kommunikationseinrichtung z.B. dem genannten mobilen Gerät zuzuleiten.

Die Darstellung der Daten kann die Darstellung von medizinischen (Bild- bzw. Patienten-)Daten umfassen.

Ein weiterer Aspekt der Erfindung ist ein zugehöriges Verfahren, das als Betriebsverfahren für die oben genannte Kommunikationseinrichtung ausgebildet sein kann, welches hardwaremäßig und/oder firmwaremäßig und/oder softwaremäßig implementiert sein kann.

Anhand von Bedieninformationen werden bestimmte Daten durch Auswahl und mittels einer ggf. vom Nutzer gewählten Darstellungsform abgeleitet und zur deren Darstellung bereitgestellt.

Die oben genannten Weiterbildungen der Erfindung in Hinblick auf die Vorrichtung bzw. Kommunikationsanordnung gelten entsprechend für das Verfahren und umgekehrt.

Ein weiterer Aspekt der Erfindung ist ein Computerprogrammprodukt mit den Merkmalen des Anspruchs 16.

Durch das Synchronisieren der unterschiedlichen Geräte wie z.B. mobile Geräte und/oder Bedienpanels werden Dokumentations- und Visualisierungsprozesse erheblich vereinfacht. Nutzer sparen Zeit, die sie aufbringen müssten, um gewünschte Daten aus dem Pool der Gesamtheit aller auf einem im Netzwerk eingebundenen Server liegenden Daten auszuwählen und aufbereitet zu bekommen. Dieses Vorgehen kann automatisiert erfolgen.

Auch der Präsentationszustand in Form eines Presentation State kann automatisiert erstellt und auf einem weiteren (i.d.R. mobilen) Gerät wieder gelesen werden. Auf vorteilhafte Weise kann somit ein aktueller Darstellungs-/Bearbeitungszustand von medizinischen Befundungen übertragen bzw. transferiert werden.

Der Transfer einer Instanz eines Presentation States kann durch Interaktion der beteiligten physikalischen Medien (Arbeitsplatzrechner und mobile Geräte), etwa durch "Blickkontakt" (Aufnahme eines graph. Codes mittels Kamera) oder "Berührung" (Auslesen/Schreiben eines aktiven Tags) erfolgen. Das stellt eine natürlichere und nachvollziehbare Aktion für den Benutzer dar. Anstelle mit Datensätzen, Suchmasken etc. wird direkt die gerade sichtbare Arbeit übertragen.

Der Presentation Service erlaubt auch bei passiven Tags durch die in eine URL kodierten Zugriffsinformationen einen Arbeitsplatzrechner zur (automatisierten) Speicherung des Presentation States anzuweisen.

Sowohl beim Initiieren des Erzeugens des Presentation States als auch für den Transfer des Darstellungszustandes kann die Standard-Peripherie von mobilen Geräten eingesetzt werden. Es sind keine baulichen Erweiterungen notwendig.

Seitens des mobilen Gerätes kann anstelle proprietärer bzw. komplexer Protokolle ausschließlich mit URLs gearbeitet werden. Entsprechende Schemas (z.B. http://server:port/path für normale Web-Anfragen) erlauben dem mobilen Gerät zugehörige Anwendungen oder auch Apps, also Browser oder DICOM-Viewer, automatisch zu starten. Die notwendigen Daten für den Abruf gewünschter Daten z.B. in Form von Dokumenten können ebenfalls der URL entnommen werden.

### Beschreibung eines oder mehrerer Ausführungsbeispiele

Weitere Vorteile, Einzelheiten und Weiterbildungen der Erfindung ergeben sich aus der nachfolgenden Beschreibung von Ausführungsbeispielen in Verbindung mit den Zeichnungen. In den Zeichnungen zeigen:
Figur 1 ein Beispiel einer Kommunikationsanordnung im erfindungsgemäßen Kontext und
Figur 2 ein beispielhaftes Ablaufdiagramm zur Übertragung von passiven bzw. aktiven Tags zwischen den beteiligten Komponenten mobilen Gerät bzw. Mobilgerät M, Arbeitsplatzrechner AP, dem Server S, umfassend den Service Serv und eine Daten speichernde Speicherstelle SP.

Korrespondierende Bezeichnungen bzw. die zugehörigen Bezugszeichen der Zeichnungen sind nachstehend in der Bezugszeichenliste aufgelistet.

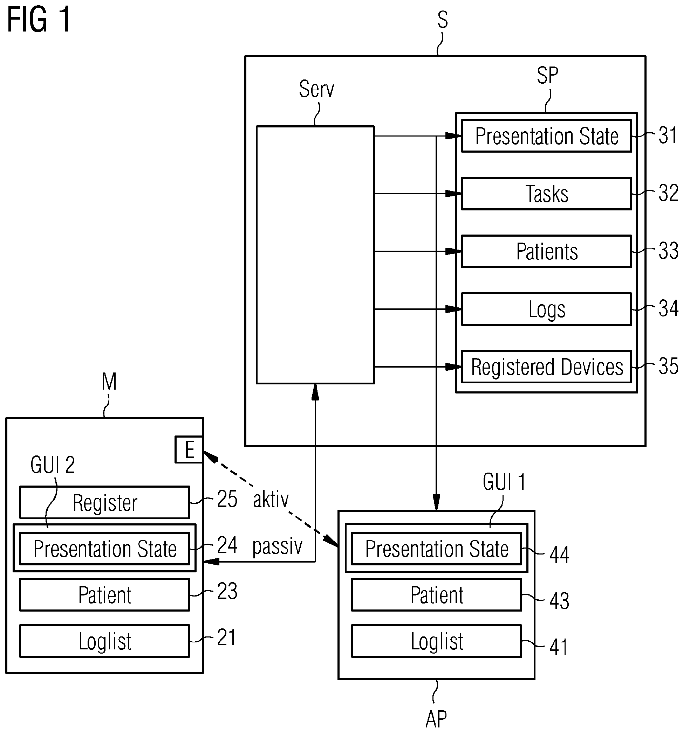

Gemäß Figur 1 sind beispielhaft folgende Komponenten bzw. Entitäten in der Kommunikationsanordnung zu finden:
- Mindestens ein (Befundungs-)Arbeitsplatzrechner AP, an dem ursprünglich gearbeitet wurde. Dort können graphische Tags angezeigt werden bzw. aktive Tags beschrieben und aktiviert werden.
- Wenigstens ein Mobilgerät M, auf welchem der letzte Darstellungszustand des Arbeitsplatzes angezeigt werden soll. Es verfügt über eine Erfassungseinheit E, z.B. eine Kamera, um graphische Tags zu erfassen, bzw. Lesegeräte für aktive Tags. Darüber hinaus kann es über eine Kommunikationsschnittstelle mit dem Netzwerk z.B. innerhalb einer medizinischen Einrichtung kommunizieren.
- Präsentation Service Serv, welcher die Arbeitsplatzrechner AP verwaltet und einen zentralen Zugriffspunkt auf diese darstellt. Der Service Serv ist von den Mobilgeräten M aus per Netzwerk erreichbar, er kann aber auch auf ein davon abgetrenntes/gesichertes Netzwerk der Arbeitsplatzrechner zugreifen.
- Eine Speicherstelle SP z.B. in Form eines Kurzzeitspeicher bzw. eines Archivs (z.B. PACS), auf welchem DICOM-Dokumente abgelegt werden können.

Nach Figur 1 können ein Arbeitsplatzrechner AP mit Bedienpanel bzw. Bildschirm, ein Mobilgerät bzw. mobile Gerät bzw. Mobile Client M über eine kabelgebundene oder drahtlose bzw. funktechnische Verbindung über ein Netzwerk mit einem Server S in Verbindung stehen. Der Arbeitsplatzrechner AP umfasst eine Bedienschnittstelle GUI1 bzw. das mobile Gerät M umfasst eine Bedienschnittstelle GUI2. Von diesen Bedienschnittstellen können Bedieninformationen durch Nutzereingaben empfangen werden. Die Geräte AP und M stehen mit einer Serviceeinheit in Verbindung, die hardware-, firmware-, middleware- und/oder softwaremäßig ausgeprägte Dienste bzw. Services Serv zur Verfügung stellt, sowie Speicherstelle bzw. Speicher SP verbunden. Der Speicher SP kann sowohl personen- bzw. patientenbezogene Daten in Form von Aufgaben 32, PatientenIDs 33 und einen oder mehrere Präsentationsstatus (PS = Presentation state) als auch gerätespezifische Daten, wie z.B. Logs 34 (Protokollierung der Datenzugriffe) und registrierte Geräte 35 speichern.

Der Server S kann beispielsweise als üblicher PC, als Workstation oder ähnliche Recheneinrichtung ausgebildet sein. Ein solcher Server S weist üblicherweise einen in der Figur 1 nicht dargestellten Prozessor P auf, der eine Applikationssoftware, eine GUI-Server-Software und eine Basissoftware umfasst. Ein Arbeitsplatzrechner AP weist in der Regel einen normalerweise als Software implementieren (GUI-)Client und eine Basissoftware auf.

Ein Server S hat die Aufgabe, die Applikationssoftware auszuführen und dabei die Bedienschnittstellen GUI1, GUI2 der Geräte z.B. ABP und M zu aktualisieren.

Bedieneingaben durch den Nutzer an einem Arbeitsplatzrechner AP können z.B. einfache Knopfbedienungen (z.B. sogenannte Single-Touch) umfassen, aber auch Gesten mit mehreren Fingern (sogenannte Multi-Touch). Anhand von Bedieninformationen, die der Nutzer eingibt, werden bestimmte Daten durch Auswahl und mittels einer ggf. vom Nutzer gewählten Darstellungsform abgeleitet und zur deren Darstellung an der Bedienschnittstelle bereitgestellt.

Ferner ist es möglich, neben den reinen Bedieneingaben auch weitere Informationen, z.B. Sicherheitsinformationen in Form eines Zustimmsignals, zu übertragen. Dies kann beispielsweise dann sinnvoll sein, wenn die Änderung des zumindest einen Inhaltes zur Auslösung einer sicherheitskritischen bzw. sicherheitsrelevanten Funktion des Geräts führt.

Die Anzeige der Daten auf dem jeweiligen Arbeitsplatzrechner AP kann durch einen entsprechenden Service gesteuert werden. Bedieneingaben, die ggf. auf einem Touchscreen eines Mobilgerätes M erfolgen, sollten gegen Ausfälle der Hardware abgesichert sein.

Falls der Server S ein PC ist, kann die Ausgabe der Bedienoberfläche nicht nur auf einen realen Bildschirm, sondern auch auf einem virtuellen Bildschirm erfolgen.

Durch die Verwendung eines Mobilgeräts M, das mit Kommunikationssensoren ausgestattet ist, die in eine Erfassungseinheit E (bspw. RFID- oder NFC-Schnittstellen, oder Kameras zum Lesen von Bar- bzw. QR-Codes) integriert sind, kann das Einlesen von Daten z.B. eine Anfrage zum Präsentationsstatus 24, PatientenID 23 automatisiert erfolgen. Es kann eine breitere Masse an Daten zusammengesammelt (bspw. Stammdaten 32 und 33) und auf verbundenen Geräten M und AP z.B. zu deren Anzeige zur Verfügung gestellt werden.

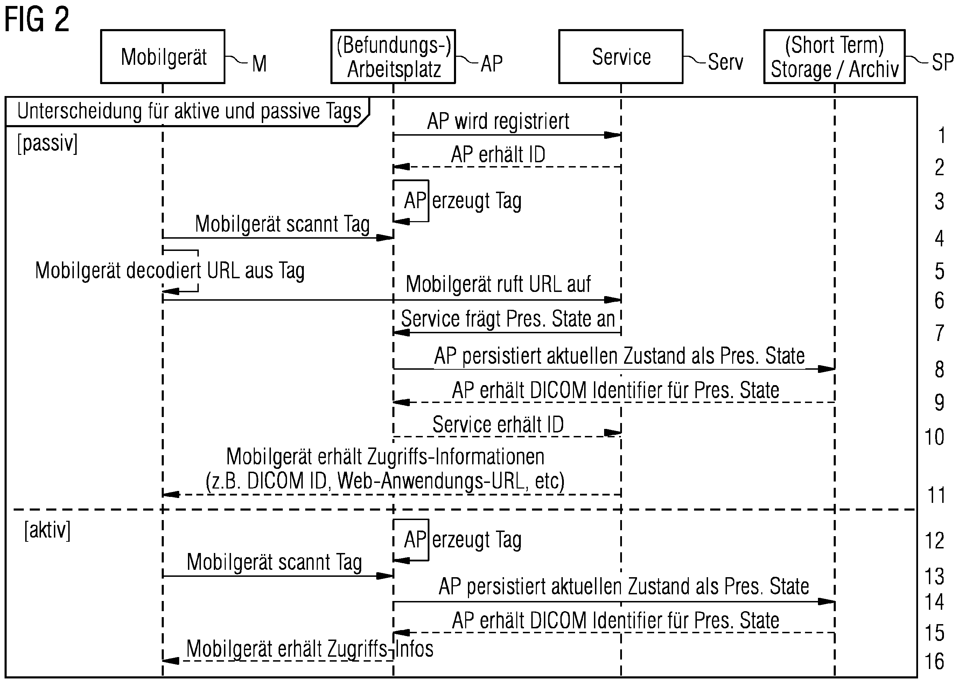

Figur 2 zeigt die oben bereits erwähnten beteiligten Entitäten des Systems bzw. der Systemanordnung und den Ablauf, um den Zustand bzw. Status auf dem Arbeitsplatzrechner als PS (Presentation State) zu persistieren bzw. festzuschreiben und die relevanten Zugriffsinformationen hierfür zu erhalten.

Das in Figur 2 gezeigte Sequenz- bzw. Ablaufdiagramm mit den dort bezeichneten Schritten 1 bis 16 unterscheidet dabei zwischen passiven und aktiven Tags. Passive Tags umfassen hierbei passive NFC-Tags aber auch graphische Codes/Tags wie QR-Codes.

In letzterem Fall wird der Präsentation Service nicht benötigt. Für eine möglichst breite Abdeckung durch Standardgeräte sollte das System jedoch beide Zugriffsarten unterstützen. Während graphische Tags von quasi allen Geräten erkannt werden können, findet Peripherie zum Lesen von passiven und aktiven NFC-Tags und/oder weiterer aktiver Tags derzeit nur in einigen gehobenen Geräteklassen Anwendung.

In Schritt 1 wird bei passiven Tags jeder Arbeitsplatzrechner AP am Präsentation Service Serv registriert. Dadurch erhält jeder Arbeitsplatzrechner eine eindeutige Identifikation (ID) .

In Schritt 2 bzw. 12 kann der Arbeitsplatzrechner daraufhin ein Tag erzeugen und zur Verfügung stellen. Das Tag trägt dabei eine URL (Uniform Resource Locator), welche den Präsentation Service adressiert. Darüber hinaus können in der URL der Arbeitsplatzrechner selbst (durch die zuvor erhaltene ID) und ggf. IDs für das aktuell dargestellte Projekt o.ä. kodiert sein. In Schritt 4 bzw. 13 scannt das Mobilgerät M das Tag und dekodiert in Schritt 5 anschließend selbständig die URL. Die URL ruft das Mobilgerät M in Schritt 6 dann auf, wobei die HTTP-Anfrage an den Präsentation Service Serv gerichtet ist. In Schritt 7 weist der Service Serv nun entsprechend der enthaltenen Informationen (Arbeitsplatzrechner, Projekt, etc.) den entsprechenden Arbeitsplatzrechner AP an, den Zustand z.B. als DICOM Presentation State zu speichern. Gemäß Schritt 8 bzw. 14 erstellt der Arbeitsplatzrechner ein PS-Dokument und persistiert dadurch den Presentation State an einer Speicherstelle SP, von welcher der Arbeitsplatzrechner eine DICOM-ID für das PS-Dokument erhält (Schritt 9 bzw. 15). In Schritt 10 gibt der Arbeitsplatzrechner diese ID an den Präsentation Service zurück, welcher sie in Schritt 11 wiederum (ggf. mit weiteren Daten für den Zugriff auf den letzten Darstellungszustand) an das Mobilgerät M weitergibt.

Bei aktiven Tags muss nicht erst eine URL aufgerufen werden, welche den Präsentation Service adressiert und anweist, beim Arbeitsplatzrechner eine PS-Persistenz anzufragen. Statt dessen kann der Arbeitsplatzrechner selbst die notwendigen Schritte initiieren.

Befindet sich der Arbeitsplatzrechner innerhalb desselben Netzwerkes wie das Mobilgerät M, kann auch bei passiven Tags (abweichend von der Darstellung in Figur 1) ebenfalls auf den Präsentation Service und die vorgelagerte Registrierung des Arbeitsplatzrechners verzichtet werden. Statt dessen wird in der URL bereits der Arbeitsplatzrechner selbst adressiert.

Das Sequenzdiagramm endet in beiden Fällen (siehe Schritt 11 bzw. 16) mit dem Erhalt relevanter Zugriffs-Informationen durch das Mobilgerät M. In diesen Informationen wird dabei i.d.R. der DICOM-Identifier für den Presentation State enthalten sein. Das Mobilgerät kann daraufhin eine geeignete App starten, um den Zustand mittels des PS wiederherzustellen. Der PS wird mittels der ID mit allen zugehörigen Dokumenten von Speicherstelle SP geladen. Alternativ oder zusätzlich können weitere Daten enthalten sein, etwa die URL zu einer Web-Anwendung, welche den Zugriff per Browser auf dem Mobilgerät M anstelle einer nativen App erlaubt. Auch die direkte Übertragung des PS-Dokuments selbst ist denkbar.

Bei einem aktiven Tag können die Nutzdaten (Payload) des gelesenen Tags diese Informationen bzw. bei einem passiven Tag die HTTP-Antwort nach Aufruf der gelesenen URL enthalten. Im Fall einer nachgelagerten Web-Anwendung könnte der Präsentation Service auch einen HTTP-Redirect (HTTP-Umleitung) auf die Web-Anwendung erzwingen. Bei einem aktiven Tag könnte die Antwort bereits die finale URL zur Web-Anwendung sein.

Anstelle eines Arbeitsplatzrechners AP kann auch ein weiteres Mobilgerät zum schon vorhandenen Mobilgerät M als Ausgangspunkt dienen. Der Ablauf ist dabei analog zum Ablauf zwischen einem Arbeitsplatzrechner AP und einem Mobilgerät M.

Ist der Ausgangspunkt ein Mobilgerät M und das Zielgerät jedoch ein stationärer Arbeitsplatzrechner AP, so kann der Ablauf ebenfalls analog zum umgekehrten Fall stattfinden. Hierbei kommen jedoch passive Tags nur bedingt in Frage, da das Scannen des Tags durch den Arbeitsplatzrechner AP vom Mobilgerät M unpraktisch wäre.

Vorteilhafter wäre es in dieser Übertragungsrichtung, wenn das Mobilgerät explizit in einen Sendemodus versetzt wird. Beim Scannen eines Tags wird dabei kein PS angefordert, sondern direkt die Zugriffsinformationen versendet. Ggf. kann der Arbeitsplatzrechner darüber hinaus in einen gesonderten Empfangsmodus versetzt werden, so dass die Empfangsbereitschaft u.U. auch in einem solchen Tag entsprechend kodiert wäre.

In den oben genannten Varianten kann auch direkt die DICOM-ID eines Presentation States (oder gar eines Ausgangsbildes) übertragen werden, ohne explizit einen gesonderten PS zu persistieren, falls der aktuelle Darstellungszustand nicht vom geladenen Ausgangsdokument abweicht.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Kommunikationsanordnung
- mit einer ersten Kommunikationseinrichtung (AP), die eine zu einer Darstellung von Daten ausgebildete erste Bedienschnittstelle (GUI1) aufweist, und die zum Senden von mindestens einer Darstellungszustandsinformation (24,44,31) ausgebildet ist,
- mit einer zweiten Kommunikationseinrichtung (M), die eine zu einer Darstellung von Daten ausgebildete zweite Bedienschnittstelle (GUI2) aufweist, und die zum Erhalten der mindestens einen Darstellungszustandsinformation (24,44,31) ausgebildet ist,
- mit Initiierungsmitteln (E), die ein Erzeugen zumindest einer Darstellungszustandsinformation (44) hinsichtlich des Zustandes der Darstellung von Daten an der ersten Bedienschnittstelle (GUI1) initiieren, wobei an der zweiten Bedienschnittstelle (GUI2) nach Erhalt der zumindest einen Darstellungszustandsinformation (44,31) der gleiche Zustand der Darstellung wie an der ersten Bedienschnittstelle (GUI1) erreichbar ist,
**dadurch gekennzeichnet,**
**dass** zur Initiierung der Erzeugung der zumindest einen Darstellungszustandsinformation (44) ein oder mehrere Tags verwendet werden.

2. Kommunikationsanordnung nach dem vorhergehenden Anspruch,
**dadurch gekennzeichnet,**
**dass** die zweite Kommunikationseinrichtung als mobiles Gerät (M) ausgebildet ist.

3. Kommunikationsanordnung nach einem der vorhergehenden Ansprüche,
**dadurch gekennzeichnet,**
**dass** die zweite Kommunikationseinrichtung (M) die Initiierungsmittel (E) umfasst, und dass die Initiierungsmittel (E) mit der ersten Kommunikationseinrichtung (AP, S) kommunizieren können, in der Mittel zum Erzeugen der zumindest einen Darstellungszustandsinformation (44) implementiert sind.

4. Kommunikationsanordnung nach einem der vorhergehenden Ansprüche,
**dadurch gekennzeichnet,**
**dass** die zumindest eine erzeugte Darstellungszustandsinformation (44, 31) in einer Speicherstelle (SP) festgeschrieben werden kann.

5. Kommunikationsanordnung nach dem vorhergehenden Anspruch,
**dadurch gekennzeichnet,**
**dass** die zweite Kommunikationseinrichtung (M) eine die Speicherstelle (SP) adressierbare Darstellungsidentifikationsinformation (ID) erhalten kann.

6. Kommunikationsanordnung nach einem der vorhergehenden Ansprüche,
**dadurch gekennzeichnet,**
**dass** die zumindest eine Darstellungszustandsinformation (31) mittels einer die Darstellungsidentifikationsinformation (ID) umfassenden Zugriffsinformation abrufbar ist.

7. Kommunikationsanordnung nach einem der vorhergehenden Ansprüche,
**dadurch gekennzeichnet,**
**dass** die Darstellung der Daten die Darstellung von medizinischen Daten umfasst.

8. Verfahren zum Übertragen von Darstellungszustandsinformationen (24, 44, 31) von einer ersten Bedienschnittstelle (GUI1) einer ersten Kommunikationseinrichtung (AP) zu einer zweiten Bedienschnittstelle (GUI2) einer zweiten Kommunikationseinrichtung (M) über ein Netzwerk,
- wobei an solch einer Bedienschnittstelle (GUI1,GUI2) zumindest eine Darstellung von Daten bereitgestellt wird,
- wobei ein Erzeugen zumindest einer Darstellungszustandsinformation (44) hinsichtlich des Zustandes der Darstellung von Daten an der ersten Bedienschnittstelle (GUI1) initiiert wird,
- wobei an einer zweiten Bedienschnittstelle (GUI2) nach Erhalt der zumindest einen Darstellungszustandsinformation (44, 31) der gleiche Zustand der Darstellung wie an der ersten Bedienschnittstelle (GUI1) erreicht wird,
**dadurch gekennzeichnet,**
**dass** zur Initiierung der Erzeugung der Darstellungszustandsinformation (44) ein oder mehrere Tags verwendet werden.

9. Verfahren nach Anspruch 8, **dadurch gekennzeichnet,**
**dass** die zweite Kommunikationseinrichtung durch ein mobiles Gerät (M) repräsentiert wird.

10. Verfahren nach Anspruch 8 oder 9,
**dadurch gekennzeichnet,**
**dass** das Initiieren des Erzeugens der zumindest einen Darstellungszustandsinformation (44) von der zweiten Kommunikationseinrichtung (M) durchgeführt wird.

11. Verfahren nach einem der Ansprüche 8 bis 10,
**dadurch gekennzeichnet,**
**dass** das Erzeugen der zumindest einen Darstellungszustandsinformation (44) von der ersten Kommunikationseinrichtung (AP) durchgeführt wird.

12. Verfahren nach einem der Ansprüche 8 bis 11, **dadurch gekennzeichnet,**
**dass** die zumindest eine Darstellungszustandsinformation (31) in einer Speicherstelle (SP) festgeschrieben wird.

13. Verfahren nach Anspruch 8 bis 12, **dadurch gekennzeichnet,**
**dass** die zweite Kommunikationseinrichtung (M) eine die Speicherstelle (SP) adressierbare Darstellungsidentifikationsinformation (ID) erhält.

14. Verfahren nach einem der Ansprüche 8 bis 13,
**dadurch gekennzeichnet,**
**dass** die zumindest eine Darstellungszustandsinformation (31) mittels einer die Darstellungsidentifikationsinformation (ID) umfassende Zugriffsinformation abgerufen wird.

15. Verfahren nach einem der Ansprüche 8 bis 14,
**dadurch gekennzeichnet,**
**dass** die Darstellung der Daten die Darstellung von medizinischen Daten umfasst.

16. Computerprogrammprodukt mit Mitteln zur Durchführung des Verfahrens nach einem der Ansprüche 8 bis 15, wenn das Computerprogrammprodukt an einer Kommunikationseinrichtung (AP,M), insbesondere nach einem der Ansprüche 1 bis 7, zur Ausführung gebracht wird.

## Claims

1. Communication assembly
- with a first communication apparatus (AP) which has a first control interface (GUI1) which is embodied for presenting data and for transmitting at least one item of presentation state information (24, 44, 31),
- with a second communication apparatus (M), which has a second control interface (GUI2) which is embodied for presenting data and for obtaining the at least one item of presentation state information (24, 44, 31),
- with initiation means (E) which initiate a generation of at least one item of presentation state information (44) with respect to the state of the presentation of data on the first control interface (GUI1), wherein after receiving the at least one item of presentation state information (44, 31) the same state of presentation as on the first control interface (GUI1) can be achieved on the second control interface (GU12), **characterised in that**
one or more tags are used to initiate the generation of the at least one item of presentation state information (44).

2. Communication assembly according to the preceding claim,
**characterised in that**
the second communication apparatus is embodied as a mobile device (M).

3. Communication assembly according to one of the preceding claims,
**characterised in that**
the second communication apparatus (M) comprises the initiation means (E) and that the initiation means (E) can communicate with the first communication apparatus (AP, S), in which means for generating the at least one item of presentation state information (44) are implemented.

4. Communication assembly according to one of the preceding claims,
**characterised in that**
the at least one generated item of presentation state information (44, 31) can be stipulated in a storage position (SP) .

5. Communication assembly according to the preceding claim,
**characterised in that**
the second communication apparatus (M) can obtain an item of presentation identification information (ID) which can address the storage position (SP).

6. Communication assembly according to one of the preceding claims,
**characterised in that**
the at least one item of presentation state information (31) can be called up by means of access information comprising the presentation identification information (ID).

7. Communication assembly according to one of the preceding claims,
**characterised in that**
the presentation of the data comprises the presentation of medical data.

8. Method for transmitting presentation state information (24, 44, 31) from a first control interface (GUI1) of a first communication apparatus (AP) to a second control interface (GUI2) of a second communication apparatus (M) by way of a network,
- wherein at least one presentation of data is provided on one such control interface (GUI1, GUI2),
- wherein a generation of at least one item of presentation state information (44) with respect to the state of the presentation of data is initiated on the first control interface (GUI1),
- wherein on a second control interface (GUI2) after receiving the at least one item of presentation state information (44, 31), the same state of presentation as on the first control interface (GUI1) is achieved,
**characterised in that**
one or more tags are used to initiate the generation of the presentation state information (44).

9. Method according to claim 8,
**characterised in that**
the second communication apparatus is represented by a mobile device (M).

10. Method according to claim 8 or 9,
**characterised in that**
the initiation of the generation of the at least one item of presentation state information (44) is carried out by the second communication apparatus (M).

11. Method according to one of claims 8 to 10,
**characterised in that**
the generation of the at least one item of presentation state information (44) is carried out by the first communication apparatus (AP).

12. Method according to one of claims 8 to 11,
**characterised in that**
the at least one item of presentation state information (31) is stipulated in a storage position (SP).

13. Method according to claim 8 to 12,
**characterised in that**
the second communication apparatus (M) obtains an item of presentation identification information (ID) which can address the storage position (SP).

14. Method according to one of claims 8 to 13,
**characterised in that**
the at least one item of presentation state information (31) is called up by means of an item of access information comprising the item of presentation identification information (ID) .

15. Method according to one of claims 8 to 14,
**characterised in that**
the presentation of data comprises the presentation of medical data.

16. Computer program product with means for carrying out the method as claimed in one of claims 8 to 15, when the computer program product is put into effect on a communication apparatus (AP, M), in particular as claimed in one of claims 1 to 7.

## Revendications

1. Agencement de communication
- comprenant un premier dispositif (AP) de communication, qui a une première interface (GUI1) de service constituée pour une représentation de données et qui est constitué pour envoyer au moins une information (24, 44, 31) d'état de représentation,
- comprenant un deuxième dispositif (M) de communication, qui a une deuxième interface (GUI2) de service constituée pour une représentation de données et qui est constitué pour recevoir la au moins une information (24, 44, 31) d'état de représentation,
- comprenant des moyens (E) de déclenchement, qui déclenchent une production d'au moins une information (44) d'état de représentation concernant l'état de la représentation de données sur la première interface (GUI1) de service, dans lequel, sur la deuxième interface (GUI2) de service, après obtention de la au moins une information (44, 31) d'état de la représentation, le même état de la représentation que sur le premier interface (GUI1) de service peut être obtenu,
**caractérisé en ce que**,
pour déclencher la production de la au moins une information (44) d'état de la représentation, on utilise une ou plusieurs étiquettes.

2. Agencement de communication suivant la revendication précédente,
**caractérisé**
**en ce que** le deuxième dispositif de communication est constitué sous la forme d'un appareil (M) mobile.

3. Agencement de communication suivant l'une des revendications précédentes,
**caractérisé**
**en ce que** le deuxième dispositif (M) de communication comprend les moyens (E) de déclenchement, et **en ce que** les moyens (E) de déclenchement peuvent communiquer avec le premier dispositif (AP, S) de communication, dans lequel des moyens de production de la au moins une information (44) d'état de la représentation sont mis en œuvre.

4. Agencement de communication suivant l'une des revendications précédentes,
**caractérisé**
**en ce que** la au moins une information (44, 31) d'état de la représentation peut être figée dans un emplacement (SP) de mémoire.

5. Agencement de communication suivant la revendication précédente,
**caractérisé**
**en ce que** le deuxième dispositif (M) de communication peut obtenir une information (ID) d'identification de la représentation pouvant être adressée dans l'emplacement (SP) de la mémoire.

6. Agencement de communication suivant l'une des revendications précédentes,
**caractérisé**
**en ce que** la au moins une information (31) d'état de la représentation peut être appelée au moyen d'une information d'accès comprenant l'information (ID) d'identification de la représentation.

7. Agencement de communication suivant l'une des revendications précédentes,
**caractérisé**
**en ce que** la représentation des données comprend la représentation de données médicales.

8. Procédé de transmission d'informations (24, 44, 31) d'état de représentation d'une première interface (GUI1) de service d'un premier dispositif (AP) de communication à une deuxième interface (GUI2) de service d'un deuxième dispositif (M) de communication, par l'intermédiaire d'un réseau,
- dans lequel on met à disposition au moins une représentation de données sur une telle interface (GUI1, GUI2) de service,
- dans lequel on déclenche une production d'au moins une information (44) d'état de la représentation concernant l'état de la représentation de données sur la première interface (GUI1) de service,
- dans lequel, sur une deuxième interface (GUI2) de service, après l'obtention de la au moins une information (44, 31) d'état de la représentation, on obtient le même état de la représentation que sur la première interface (GUI1) de service,
**caractérisé**
**en ce que**, pour déclencher la production de l'information (44) d'état de la représentation, on utilise une ou plusieurs étiquettes.

9. Procédé suivant la revendication 8,
**caractérisé**
**en ce que** l'on représente le deuxième dispositif de communication par un appareil (M) mobile.

10. Procédé suivant la revendication 8 ou 9,
**caractérisé**
**en ce que** l'on effectue le déclenchement de la production de la au moins une information (44) d'état de la représentation par le deuxième dispositif (M) de communication.

11. Procédé suivant l'une des revendications 8 à 10,
**caractérisé**
**en ce que** l'on effectue la production de la au moins une information (44) d'état de la représentation par le premier dispositif (AP) de communication.

12. Procédé suivant l'une des revendications 8 à 11,
**caractérisé**
**en ce que** l'on fige la au moins une information (3) d'état de la représentation dans un emplacement (SP) de mémoire.

13. Procédé suivant la revendication 8 à 12,
**caractérisé**
**en ce que** le deuxième dispositif (M) de communication reçoit une information (ID) d'identification de la représentation pouvant être adressée dans l'emplacement (SP) de mémoire.

14. Procédé suivant l'une des revendications 8 à 13,
**caractérisé**
**en ce que** l'on appelle la au moins une information (31) d'état de la représentation au moyen d'une information d'accès comprenant l'information (ID) d'identification de la représentation.

15. Procédé suivant l'une des revendications 8 à 14,
**caractérisé**
**en ce que** la représentation des données comprend la représentation de données médicales.

16. Produit de programme d'ordinateur ayant des moyens pour effectuer le procédé suivant l'une des revendications 8 à 15, lorsque le produit de programme d'ordinateur est mis en réalisation sur un dispositif (AP, M) de communication, notamment suivant l'une des revendications 1 à 7.
